# EUROPEAN PATENT APPLICATION

(11) **EP 2 322 038 A1**
(43) Date of publication of application: **18.05.2011**
(21) Application number: 09800216.5
(22) Date of filing: 22.07.2009
(51) Int. Cl.: A01N 31/16, A01N 25/02, A01N 31/08, A01N 37/10, A01P 3/00, A61K 8/34, A61K 8/37

(54) **METHOD FOR PRODUCING ANTIBACTERIAL AGENT-CONTAINING LIQUID**

(30) Priority: 23.07.2008 JP 2008189614
(71) Applicant: Kao Corporation, Chuo-ku Tokyo 103-8210 (JP)
(72) Inventor: MATSUYAMA, Kazuo, Wakayama-shi Wakayama 640-8580 (JP); MINE, Koji, Wakayama-shi Wakayama 640-8580 (JP); KUBOTA, Hiromi, Haga-gun Tochigi 321-3497 (JP); YANO, Takehisa, Haga-gun Tochigi 321-3497 (JP)
(74) Representative: HOFFMANN EITLE
(86) International application number: PCT/JP2009/003451
(87) International publication number: WO 2010/010700

(57) **Abstract**

A method for producing an antimicrobial-containing solution includes mixing an alkaline aqueous solution which dissolves a phenol-based antimicrobial, and has a pH of 9 or higher and acid which has a pH 6 or lower.

## Description

### TECHNICAL FIELD

The present invention relates to a method for producing an antimicrobial-containing solution added to household products, toiletry, cosmetics, etc.

### BACKGROUND ART

Chlorophenol-based antimicrobials, parabens, and isopropylmethylphenol, which are phenol-based antimicrobials, have been known as fine antimicrobials. Most of these phenol-based antimicrobials are solid at normal temperature, and is almost insoluble in water. Therefore, in most cases, the phenol-based antimicrobials are generally blended in a detergent containing a surfactant, or are dissolved in an oily ingredient or an organic solvent.

Patent Document 1 teaches a method for producing a solution having a concentration sufficient to be pharmacologically effective by dissolving desfluoro pyrrolidone carboxylic acid compound or salt thereof having antimicrobial activity in an aqueous solution having a pH of 10 or higher, and adding acid to the obtained solution in the presence of cyclodextrin and magnesium salt to adjust the pH to 6-8, i.e., almost neutral, thereby improving solubility of the compound.

### CITATION LIST

### PATENT DOCUMENT

[Patent Document 1] Japanese Patent Publication No. 2003-226643

### SUMMARY OF THE INVENTION

A method for producing an antimicrobial-containing solution of the present invention includes: mixing an alkaline aqueous solution which dissolves a phenol-based antimicrobial, and has a pH of 9 or higher and acid which has a pH of 6 or lower.

Another method for producing an antimicrobial-containing solution of the present invention is a method for producing an antimicrobial-containing solution containing fine particles of a phenol-based antimicrobial having an average particle diameter of 50-5000 nm obtained by a dynamic light scattering measurement, the method including: placing an alkaline aqueous solution which dissolves a phenol-based antimicrobial, and has pH of 9 or higher in a first chamber of a container having a solution discharging part, and placing acid having a pH of 6 or lower in a second chamber of the container having a solution discharging part; simultaneously discharging the alkaline aqueous solution from the solution discharging part of the first chamber of the container, and the acid from the solution discharging part of the second chamber of the container to mix the alkaline aqueous solution and the acid outside the container.

### BRIEF DESCRIPTION OF THE DRAWINGS

[FIG. 1] FIG. 1 is a cross-sectional view of a mixer.
[FIG. 2] FIG. 2 is a perspective view of a container.
[FIG. 3] FIG. 3 is a view illustrating a method for producing an antimicrobial-containing solution.

### DESCRIPTION OF EMBODIMENTS

An embodiment will be descried in detail below.

A method for producing an antimicrobial-containing solution of the present embodiment includes mixing an alkaline aqueous solution which dissolves a phenol-based antimicrobial, and has a pH of 9 or higher and acid which has a pH of 6 or lower.

When blended in a detergent, a phenol-based antimicrobial which is solid at normal temperature, and is almost insoluble in water (e.g., chlorophenol-based antimicrobials, parabens, isopropylmethylphenol, etc.) decreases in antimicrobial and bactericidal properties in many cases due to the presence of a surfactant. In order to obtain sufficient antimicrobial and bactericidal properties, the content of the phenol-based antimicrobial blended in the detergent has to be increased. On the other hand, the content of the phenol-based antimicrobial in household products, toiletry, cosmetics, etc. is controlled in view of safety in many cases. Further, the range of application of the phenol-based antimicrobial is limited when the phenol-based antimicrobial is dissolved in an oily ingredient or an organic solvent.

According to the method for producing the antimicrobial-containing solution of the present embodiment, an alkaline aqueous solution which dissolves a phenol-based antimicrobial, and has a pH of 9 or higher and acid which has a pH of 6 or lower are mixed. This allows preparation of a water-based antimicrobial-containing solution which exerts a great antimicrobial property even when a concentration of a phenol-based antimicrobial is too low to exert the antimicrobial property according to the conventional technology.

Examples of the phenol-based antimicrobial include chlorophenol-based antimicrobials, parabens, isopropylmethylphenol, etc. The phenol-based antimicrobial may be a single compound, or may be two or more compounds. The concentration of the phenol-based antimicrobial in the alkaline aqueous solution is preferably 10-5000 mg/L, more preferably 20-3000 mg/L.

The phenol-based antimicrobial is preferably solid at normal temperature, and is almost insoluble in water. In the present application, the term "solid at normal temperature" indicates that a melting point of a simple substance is 35°C or higher. The term "almost insoluble in water" indicates that solubility in water at 20°C is of 3000 mg/L or lower.

As specific examples of the phenol-based antimicrobial, the chlorophenol-based antimicrobials include, for examples, triclosan(5-chloro-2-[2,4-dichlorophenoxyl]phenol), chlorothymol, carvacrol, chlorophene, dichlorophene, hexachlorophene, chloroxylenol, chlorocresol, etc. Among them, triclosan is particularly preferable. The parabens include, for example, methyl parahydroxybenzoate, ethyl parahydroxybenzoate, propyl parahydroxybenzoate, isopropyl parahydroxybenzoate, butyl parahydroxybenzoate, isobutyl parahydroxybenzoate, benzyl parahydroxybenzoate, etc. Among them, propyl parahydroxybenzoate is particularly preferable. Other examples of the phenol-based antimicrobial include o-phenylphenol, 4-isopropylmethylphenol, etc.

The alkaline aqueous solution may be, for example, an aqueous solution of an alkaline agent, such as sodium hydroxide, potassium hydroxide, sodium carbonate, potassium carbonate, sodium bicarbonate, potassium bicarbonate, trisodium phosphate, etc., and in which the phenol-based antimicrobial- is dissolved. The alkaline agent contained in the alkaline aqueous solution may be a single substance, or may be two or more substances.

The alkaline aqueous solution has a pH of 9 or higher, preferably 10 or higher. The alkaline aqueous solution preferably has a pH of 14 or lower, more preferably 13 or lower.

The alkaline aqueous solution may contain polyvinylpyrrolidone to improve stability of the obtained antimicrobial-containing solution during storage. Polyvinylpyrrolidone may preferably be contained in the mass ratio of 0.1-0.5 relative to the content of the phenol-based antimicrobial.

The alkaline aqueous solution may contain a surfactant and/or an organic solvent to such a degree that the surfactant and the organic solvent do not greatly affect the antimicrobial property of the phenol-based antimicrobial. Examples of the surfactant include, for example, anionic surfactants such as sodium dodecyl sulfate, sodium polyoxyethylene alkyl ether sulfate, methyl lauroyl taurate, etc. Examples of the organic solvent include, for example, water-miscible ethanol, dimethyl sulfoxide, dimethylformamide, dimethylacetamide, glycerin, propylene glycol, etc. Preferably, the alkaline aqueous solution does not substantially contain the surfactant and the organic solvent.

The acid to be mixed with the alkaline aqueous solution may be liquid such as an acidic aqueous solution, or water-soluble solid.

The acid may be an inorganic acid, or an organic acid. Specifically, examples of the inorganic acid include hydrochloric acid (diluted hydrochloric acid), sulfuric acid (diluted sulfuric acid), phosphoric acid, etc. Examples of the organic acid include, for example, acetic acid, citric acid, etc. The acid may be a single type of acid, or may be two or more types of acids.

The acid preferably has a pH of 6 or lower, preferably 1-6, more preferably 2-5.

Like the alkaline aqueous solution, the acid may contain polyvinylpyrrolidone to improve stability of the obtained antimicrobial-containing solution during storage. Polyvinylpyrrolidone may be contained in the mass ratio of 0.1-0.5 relative to the content of the phenol-based antimicrobial.

The acid may be an acidic buffer solution. Examples of the acidic buffer solution include, for example, an aqueous solution of Bis-Tris (bis(2-hydroxyethyl)iminotris(hydroxymethyl)methane), an aqueous solution of HEPES (2-[4-(2-Hydroxyethyl)-1-piperazinyl]ethanesulfonic acid), an aqueous solution of sodium dihydrogenphosphate, an aqueous solution of citric acid and disodium phosphate, etc.

Like the alkaline aqueous solution, the acid may contain a surfactant and/or an organic solvent to such a degree that the surfactant and the organic solvent do not greatly affect the antimicrobial property of the phenol-based antimicrobial.

The alkaline aqueous solution and the acid may be mixed by batch mixing, or continuous mixing.

The batch mixing may be performed by placing one of the alkaline aqueous solution and the acid in a mixing tank, adding the other little by little in the tank, and stirring them with a stirring blade such as a propeller blade, an anchor blade, a homomixer, etc. The acid may be added to the alkaline aqueous solution, or the alkaline aqueous solution may be added to the acid.

The continuous mixing may be performed by introducing the alkaline aqueous solution and the acid in a merged state, and allowing the alkaline aqueous solution and the acid to flow in a mixer to be mixed together. The mixer used for the continuous mixing may be, for example, a static mixer, a line mixer, a micro mixer, etc.

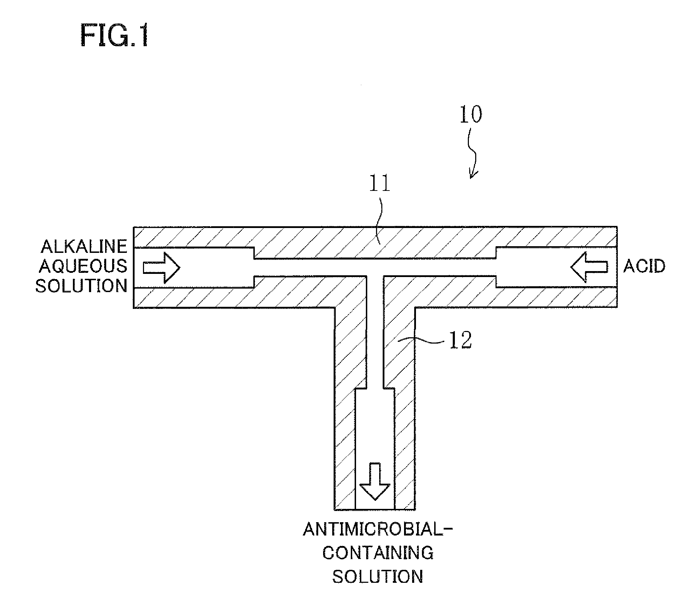

Alternatively, the continuous mixing may be performed by introducing the alkaline aqueous solution and the acid independently in the mixer, and allowing the alkaline aqueous solution and the acid to flow in the mixer to be mixed together. Specifically, for example, as shown in FIG. 1, the alkaline aqueous solution is introduced in a T-shaped mixer 10 from one end of a straight part 11, and the acid is introduced in the T-shaped mixer 10 from the other end of the straight part 11 to allow the alkaline aqueous solution and the acid to meet at the center. Then, the obtained flow is introduced to a branch part 12 (e.g., a diameter of a flow path of a narrowed part is 0.1-0.5 mm, and a length of the flow path is 0.3-3 mm) at a total flow rate of 1-100 ml/min.

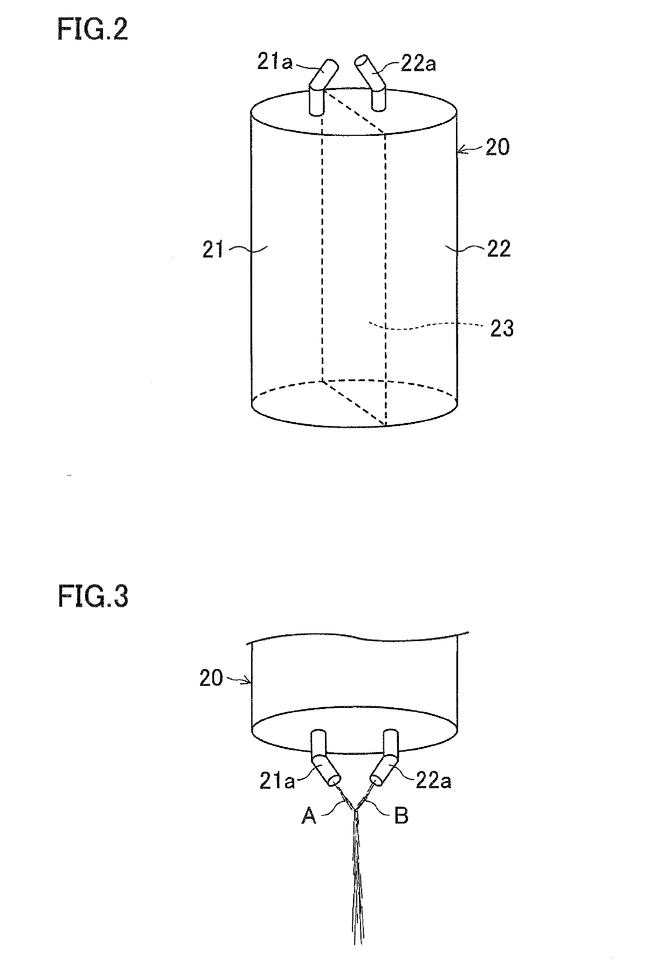

Further, the continuous mixing may be performed using a container 20 having a first chamber 21 and a second chamber 22 which are divided by an internal divider 23, and have nozzles (solution discharging parts) 21 a, 22a as shown in FIG. 2. Specifically, the alkaline aqueous solution which contains the phenol-based antimicrobials, and has a pH of 9 or higher is placed in the first chamber 21 of the container 20, and the acid having a pH of 6 or lower is placed in the second chamber 22 of the container 20. Then, the container 20 is compressed to simultaneously discharge the alkaline aqueous solution A from the nozzle 21 a of the first chamber, and the acid B from the nozzle 22a of the second chamber, thereby allowing the solutions to meet outside the container 20 to be mixed together as shown in FIG. 3.

The alkaline aqueous solution and the acid are preferably mixed in a volume mixing ratio of alkaline aqueous solution/acid = 1/400-100/1.

The produced antimicrobial-containing solution may contain the antimicrobial in a concentration lower than, or not lower than the solubility of the phenol-based antimicrobial in a mixed aqueous solution of the alkaline aqueous solution and the acid. When the antimicrobial concentration is lower than the solubility of the phenol-based antimicrobial, the antimicrobial-containing solution is an antimicrobial solution in which the phenol-based antimicrobial is dissolved in a water-based solvent having water as a main ingredient. When the antimicrobial concentration is not lower than the solubility of the phenol-based antimicrobial, the antimicrobial-containing solution is an antimicrobial dispersion in which fine particles of the phenol-based antimicrobial are precipitated, and are dispersed in a water-based dispersion medium having water as a main ingredient. In the antimicrobial dispersion, an average particle diameter of the fine particles of the phenol-based antimicrobial is preferably 50-5000 nm. The average particle diameter of the fine particles of the phenol-based antimicrobial is obtained by a dynamic light scattering measurement.

The produced antimicrobial-containing solution preferably has a pH of 4-9, more preferably 5-8 in order to obtain an effective antimicrobial- property, and ease of handling in use.

The produced antimicrobial-containing solution preferably has a suitable concentration of the phenol-based antimicrobial depending on the type of the phenol-based antimicrobial in order to show an effective antimicrobial property, and to reduce coagulation of the precipitated particles. For example, when the phenol-based antimicrobial is triclosan, the concentration of the phenol-based antimicrobial in the antimicrobial-containing solution is preferably 8-200 mg/L, preferably 10-150 mg/L. From the conventional expertise, such a concentration of the phenol-based antimicrobial is not sufficient to show the antimicrobial properly. However, according to the method for producing the antimicrobial-containing solution of the present embodiment, a water-based antimicrobial-containing solution which shows a great antimicrobial property even when the concentration of the phenol-based antimicrobial is too low to show the antimicrobial property according to the conventional technology can be produced by mixing the alkaline aqueous solution which dissolves the phenol-based antimicrobial, and has a pH of 9 or higher and the acid having a pH of 6 or lower.

To the produced antimicrobial-containing solution, a preservative, a viscous material, various types of polymers, etc. may be added to such a degree that these additives do not greatly affect the antimicrobial properties of the phenol-based antimicrobial. The produced antimicrobial-containing solution is added to household products, toiletry, cosmetics, etc.

### [Examples]

### [First Evaluation Test]

### (Antimicrobial-Containing Solution)

### <Example 1>

An alkaline aqueous solution having a triclosan concentration of 200 mg/L (solvent: alkaline water, NaOH concentration: 0.02 mol/L, pH:12) was fed to a T-shaped mixer (a micro mixer made of SUS316, and has an inner flow path diameter of 0.15 mm) of the same structure as that shown in FIG. 1 from an end of a straight part of the T-shaped mixer by a syringe pump, and an acidic buffer solution, i.e., a HEPES aqueous solution (HEPES concentration: 0.1 mol/L, pH: S.T), was fed to the T-shaped mixer from the other end of the straight part by a syringe pump at room temperature (20°C) in a volume mixing ratio of alkaline aqueous solution/HEPES aqueous solution = 1/1 at a total flow rate of 6 ml/min. Then, an antimicrobial-containing solution of Example 1 was collected from a branched part.

The antimicrobial-containing solution of Example 1 had a triclosan concentration of 100 mg/L, and a pH of 7.0.

### <Example 2>

An antimicrobial-containing solution of Example 2 was obtained in the same manner as Example 1 except that an alkaline aqueous solution having a triclosan concentration of 50 mg/L was used.

The antimicrobial-containing solution of Example 2 had a triclosan concentration of 25 mg/L, and a pH of 7.0.

### <Example 3>

An antimicrobial-containing solution of Example 3 was obtained in the same manner as Example 1 except that an alkaline aqueous solution having a triclosan concentration of 24 mg/L was used.

The antimicrobial-containing solution of Example 3 had a triclosan concentration of 12 mg/L, and a pH of 7.0.

### <Example 4>

An antimicrobial-containing solution of Example 4 was obtained in the same manner as Example 1 except that an alkaline aqueous solution having a triclosan concentration of 20 mg/L was used.

The antimicrobial-containing solution of Example 4 had a triclosan concentration of 10 mg/L, and a pH of 7.0.

### <Example 5>

An antimicrobial-containing solution was obtained in the same manner as Example 1 except that an alkaline aqueous solution having a triclosan concentration of 800 mg/L (solvent: alkaline water, NaOH concentration: 0.06 mol/L, pH: 13) was used, an acidic buffer solution, i.e., a sodium dihydrogenphosphate aqueous solution (sodium dihydrogenphosphate concentration: 0.1 mol/L, pH: 4.4) was used as the acid, and the obtained product was diluted 4-fold with water. Thus, an antimicrobial-containing solution of Example 5 was obtained.

The antimicrobial-containing solution of Example 5 had a triclosan concentration of 100 mg/L, and a pH of 7.0.

### <Example 6>

An alkaline aqueous solution having a triclosan concentration of 200 mg/L (solvent: alkaline water, NaHCO₃ concentration: 0.04 mol/L, Na₂CO₃ concentration: 0.06 mol/L, pH: 10.3) was added to a 0.05 mol/L citric acid aqueous solution (pH: 2.1) in a test tube at room temperature (20°C) in a volume mixing ratio of alkaline aqueous solution/citric acid aqueous solution =1/1. Thus, an antimicrobial-containing solution of Example 6 was obtained in the test tube.

The antimicrobial-containing solution of Example 6 had a triclosan concentration of 100 mg/L, and a pH of 5.9.

### <Example 7>

An alkaline aqueous solution having a triclosan concentration of 10000 mg/L (solvent: alkaline water, NaOH concentration: 1 mol/L, pH: 13.6) was added to a 0.05 mol/L HEPES aqueous solution (pH: 5.2) in a test tube at room temperature (20°C) in a volume mixing ratio of alkaline aqueous solution/HEPES aqueous solution = 1/99. Thus, an antimicrobial-containing solution of Example 7 was obtained in the test tube.

The antimicrobial-containing solution of Example 7 had a triclosan concentration of 100 mg/L, and a pH of 7.7.

### <Example 8>

To an alkaline aqueous solution having a triclosan concentration of 100 mg/L (solvent: alkaline water, NaHCO₃ concentration: 0.02 mol/L, Na₂CO₃ concentration: 0.03 mol/L, pH: 10.3) in a test tube, 1 mol/L sulfuric acid (pH: 0.5) was added in a volume mixing ratio of alkaline aqueous solution/sulfuric acid = 49/1 at room temperature (20°C). Thus, an antimicrobial-containing solution of Example 8 was obtained in the test tube.

The antimicrobial-containing solution of Example 8 had a triclosan concentration of 100 mg/L, and a pH of 6.7.

### <Example 9>

An alkaline aqueous solution having a triclosan concentration of 200 mg/L (solvent: alkaline water, trisodium phosphate concentration: 0.1 mol/L, pH: 12.6) was added to a 0.05 mol/L citric acid aqueous solution (pH: 2.1) in a test tube at room temperature (20°C) in a volume mixing ratio of alkaline aqueous solution/citric acid aqueous solution =1/1. Thus, an antimicrobial-containing solution of Example 9 was obtained in the test tube.

The antimicrobial-containing solution of Example 9 had a triclosan concentration of 100 mg/L, and a pH of 7.0.

### <Example 10>

An alkaline aqueous solution having a triclosan concentration of 800 mg/L (solvent: alkaline water, NaOH concentration: 0.02 mol/L, pH: 12) was placed in a first chamber of a container of the same structure as that shown in FIG. 2, and a 0.1 mol/L HEPES aqueous solution (pH: 5.1) was placed in a second chamber of the container. The alkaline aqueous solution was discharged from a nozzle of the first chamber, and the HEPES aqueous solution was discharged from a nozzle of the second chamber simultaneously to allow the alkaline aqueous solution and the HEPES aqueous solution to meet outside the container, thereby mixing the alkaline aqueous solution and the HEPES aqueous solution in a volume mixing ratio of alkaline aqueous solution/HEPES aqueous solution = 1/1. Thus, an antimicrobial-containing solution of Example 10 was obtained.

The antimicrobial-containing solution of Example 10 had a triclosan concentration of 400 mg/L, and a pH of 7.0.

### comparative Example 1>

An aqueous solution having a HEPES concentration of 0.05 mol/L, and a NaOH concentration of 0.01 mol/L was added to an ethanol solution having a triclosan concentration of 40 g/L in a test tube at room temperature (20°C) in a volume mixing ratio of ethanol solution/aqueous solution = 1/399. Thus, an antimicrobial-containing solution of Comparative Example 1 was obtained in the test tube.

The antimicrobial-containing solution of Comparative Example 1 had a triclosan concentration of 100 mg/L, and a pH of 7.0.

### <Comparative Example 2>

An antimicrobial-containing solution of Comparative Example 2 was obtained in the same manner as Comparative Example 1 except that the ethanol solution containing triclosan was replaced with a propylene glycol solution having a triclosan concentration of 40 g/L.

The antimicrobial-containing solution of Comparative Example 2 had a triclosan concentration of 100 mg/L, and a pH of 7.0.

### (Evaluation Test Method)

### <Antimicrobial Property Test>

One platinum loop of Escherichia coli NBRC3972 which was cultivated at 30°C for a night on a flat plate of soybean casein digest agar (SCDA) (manufactured by Wako Pure Chemical Industries, Ltd.) was suspended in sterilized physiological saline to prepare an inoculum.

To 1. ml of each of the antimicrobial-containing solutions of Examples 1-10 and Comparative Examples 1-2, 10 µL of the inoculum was added to incubate the inoculum and the antimicrobial-containing solution for 10 minutes. Then, after the incubation, a viable cell count was measured by a plate method. The antimicrobial-containing solution was evaluated as having the antimicrobial property: ○ when a logarithmic value of reduction in viable cell count (Log reduction value) was 2 or higher, or was evaluated as not having the antimicrobial property: × when the logarithmic value was lower than 2.

### <Presence/Absence of Particles, Measurement of Particle Diameter>

On each of the antimicrobial-containing solutions of Examples 1-10 and Comparative Examples 1-2, check of whether particle precipitation had occurred or not, and measurement of an average particle diameter were performed by a dynamic light scattering measurement using a zeta-potential & particle size analyzer (ELSZ-2 manufactured by Otsuka Electronics Co., Ltd.)

### (Results of Evaluation Test)

Tables 1-3 show the test results.

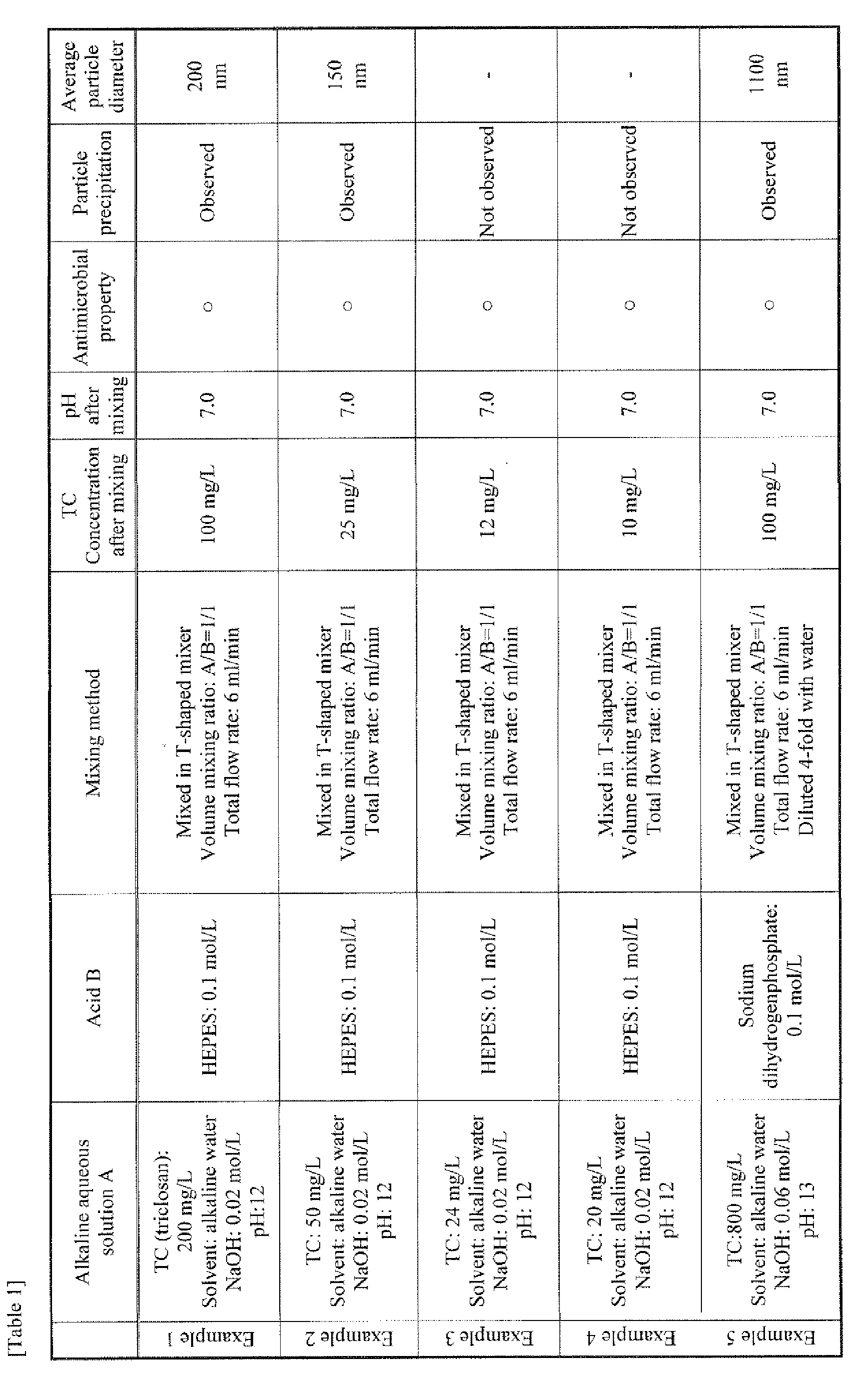

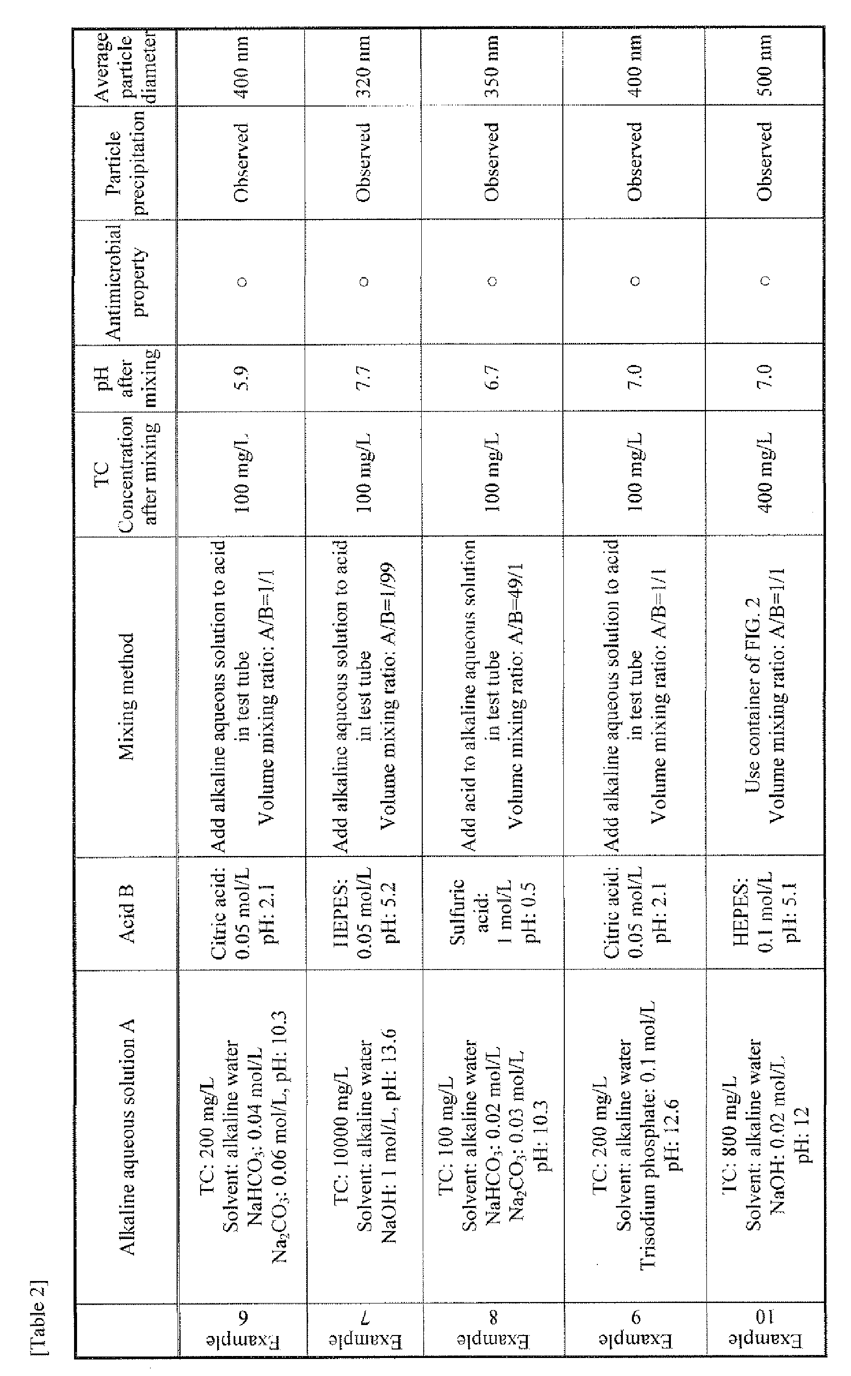

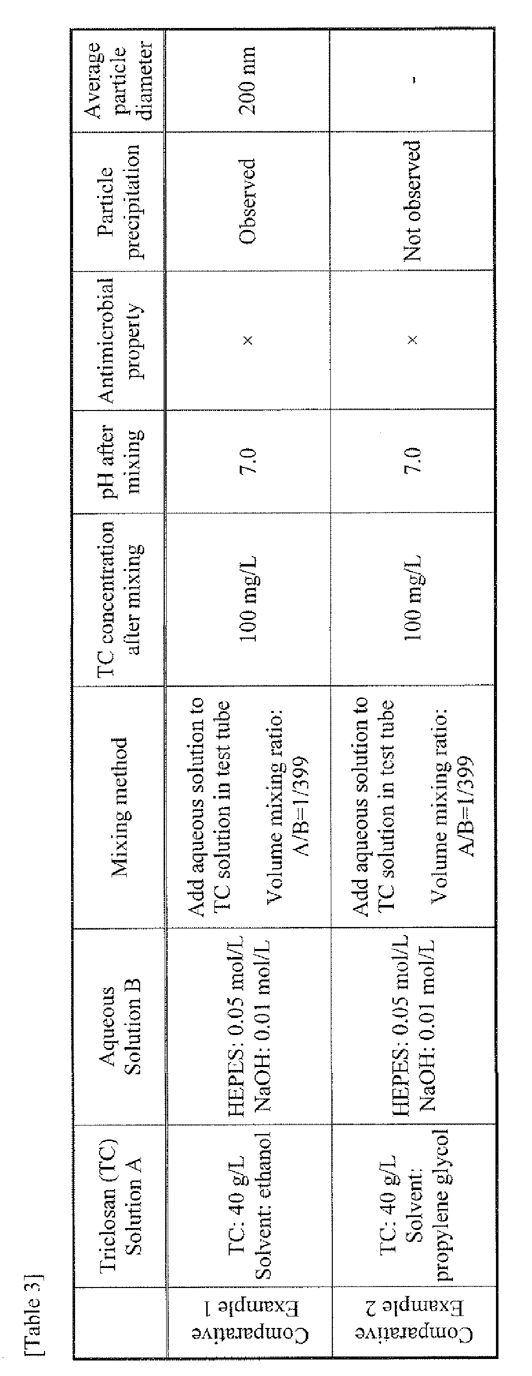

Tables 1-3 indicate that the solutions of Comparative Examples 1-2 did not show the antimicrobial property, while the solutions of Examples 1-10 showed significant antimicrobial- property even when the concentration of triclosan was significantly low as compared with the solutions of Comparative Examples 1 and 2. Although the reason for this phenomenon is not clear, it is presumed that the state of triclosan contained in the antimicrobial-containing solutions of Examples 1-10 obtained by changing the pH values is specific as compared with Comparative Example 1-2.

The solutions of Example 1, and 5-9 having the triclosan concentration of 100 mg/L, the solution of Example 2 having the triclosan concentration of 25 mg/L, and the solution of Example 10 having the triclosan concentration of 400 mg/L were antimicrobial dispersions. The solution of Example 3 having the triclosan concentration of 12 mg/L, and the solution of Example 4 having the triclosan concentration of 10 mg/L were antimicrobial solutions. The solute of Example 1 had an average particle diameter of 200 nm. The solute of Example 2 had an average particle diameter of 150 nm. The solute of Example 5 had an average particle diameter of 1100 nm. The solute of Example 6 had an average particle diameter of 400 nm. The solute of Example 7 had an average particle diameter of 320 nm. The solute of Example 8 had an average particle diameter of 350 nm. The solute of Example 9 had an average particle diameter of 400 nm. The solute of Example 10 had an average particle diameter of 500 nm. The solution of Comparative Example 1, which was an ethanol solution, was an antimicrobial dispersion, while the solution of Comparative Example 2, which was a propylene glycol solution, was an antimicrobial solution. The solute of Comparative Example 1 had an average particle diameter of 200 nm.

### [Second Evaluation Test]

### (Antimicrobial-Containing Solution)

### <Example 11>

In a 100mL volumetric flash, 6 mL of a 1 mol/L sodium hydroxide (NaOH) aqueous solution (manufactured by Kishida Chemical Co., Ltd.), 80 mg of triclosan (Ciba Specialty Chemicals Inc.), and 28 mg of polyvinylpyrrolidone (ISP: PVP-K30, weight average molecular weight: 60000) were placed, and water was added to 100 mL, thereby preparing an alkaline aqueous solution in which triclosan and polyvinylpyrrolidone were dissolved. The alkaline aqueous solution had a pH of 13. In this alkaline aqueous solution, the concentrations of sodium hydroxide, triclosan, and polyvinylpyrrolidone were 60 mmol/L, 800 mg/L, and 100 mg/L, respectively, and the mass ratio of polyvinylpyrrolidone/triclosan was 0.35. An acidic aqueous solution was prepared by dissolving sodium dihydrogenphosphate (manufactured by Wako Pure Chemical Industries, Ltd.) in water to a concentration of 100 mmol/L. The acidic aqueous solution had a pH of 4.4. The alkaline aqueous solution and the acidic aqueous solution were adjusted to 20°C.

The alkaline aqueous solution was fed to a T-shaped mixer of the same structure as that shown in FIG. 1 (a micromixer having a branch part having a cylindrical hole of 0.15 mm in diameter, and 1.0 mm in length) from an end of the straight part by a syringe pump, and the acidic aqueous solution was fed to the T-shaped mixer from the other end by a syringe pump at room temperature (20°C) in a volume mixing ratio of alkaline aqueous solution/acidic aqueous solution = 1/1 at a total flow rate of 6 ml/min. Thus, an antimicrobial-containing solution of Example 11 was collected from the branch part. The antimicrobial-containing solution of Example 11 had a triclosan concentration of 400 mg/L.

### (Evaluation Test Method)

An average particle diameter of the solution of Example 11 was obtained by a dynamic light scattering measurement using a zeta potential & particle size analyzer (ELSZ-2 manufactured by Otsuka Electronics Co., Ltd.), and visual inspection was performed (immediately after the preparation, and after one-day storage at room temperature).

### (Results of Evaluation Test)

Table 4 shows the test results.

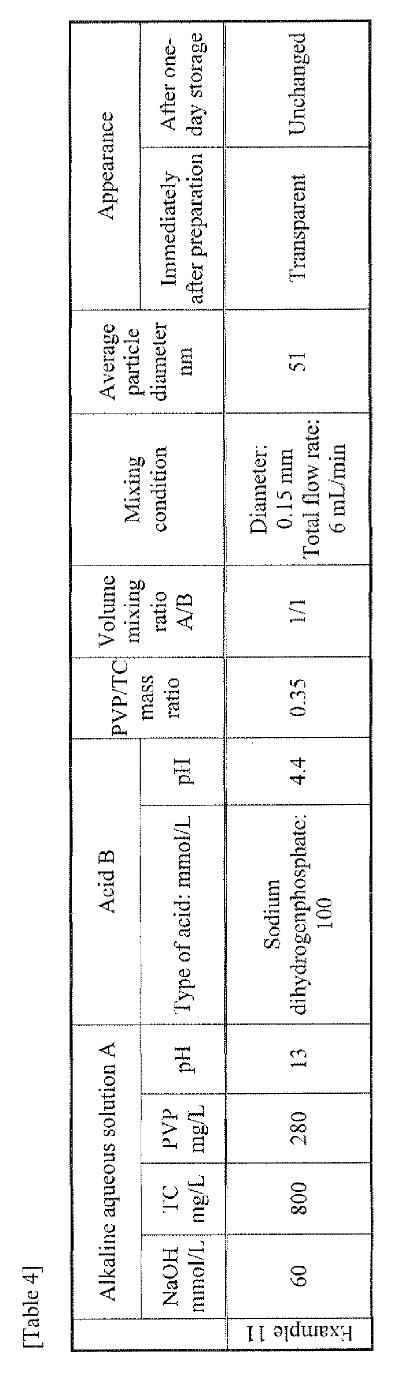

Table 4 shows that the solution of Example 11 had an average particle diameter of 51 nm. The appearance of the solution of Example 11 was transparent immediately after the preparation, and was unchanged after one-day storage.

### [Third Evaluation Test]

### (Preparation Example: Preparation of Antimicrobial-Containing Solution (Triclosan Dispersion))

### (1) Preparation of Solutions A-C

### Solution A: Alkaline Aqueous Solution Having Triclosan Concentration of 400 mg/L

In a 100mL volumetric flask, 40 mg of triclosan (manufactured by Ciba Specialty Chemicals Inc.) was placed, 20 mL of a 0.1 mol/L sodium hydroxide aqueous solution (manufactured by Kishida Chemical Co., Ltd.) was added to dissolve triclosan, and ion-exchanged water was added to 100 mL. The obtained alkaline aqueous solution having a triclosan concentration of 400 mg/L was referred to as Solution A. Solution A had a pH of 12.

### Solution B: HEPES Aqueous Solution

In a 100ml volumetric flask, 2.383 g of HEPES (manufactured by Wako Pure Chemical Industries, Ltd.) was placed, and ion-exchanged water was added to 100 mL to dissolve HEPES. The obtained HEPES aqueous solution was referred to as Solution B. Solution B had a pH of 5.1.

### Solution C: HEPES Buffer Solution Having HEPES Concentration of 0,05mol/L

In a 100 mL volumetric flask, 2.383 g of HEPES was placed, and ion-exchanged water was added to 100 mL to dissolve HEPES. In another 100 mL volumetric flask, 20 mL of a 0.1 mol/L sodium hydroxide aqueous solution was placed, and ion-exchanged water was added to 100 mL to dilute the solution. These products were mixed in a beaker to prepare a HERPES buffer solution having a HEPES concentration of 0.05 mol/L as Solution C. Solution C had a pH of 7.

### (2) Apparatus for Preparing Antimicrobial-Containing Solution

Three 30 mL plastic syringes were prepared for solutions A, B, and C, and a membrane filter having a pore diameter of 0.22 µm was attached to an end of each of the syringes. Then, the plastic syringes were attached to micro syringe pumps (manufactured by KD Scientific Inc.). PTFE tubes of 20 cm in length, and 0.8 mm in inner diameter were connected to the outlets of the syringes (filter outlet). A tube for Solution A and a tube for Solution B were connected to an opposing pair of couplings of a T-shaped joint (GL Sciences Inc.) of 0.15 mm in inner diameter in such a manner that Solutions A and B meet each other. An end of a PTFE tube of 10 cm in length, and 0.8 mm in inner diameter, was connected to the remaining end of the T-shaped joint, and the other end of the PTFE tube and a tube for Solution C were connected to an opposing pair of couplings of a T-shaped joint (Swagelok Company) of 1.3 mm in inner diameter in such a manner that a mixture of Solutions A and B and Solution C meet each other. An end of a PTFE tube of 10 cm in length, and 0.8 mm in inner diameter was connected to the remaining end of the T-shaped joint, and the other end of the PTFE tube is used as a sampling port.

### (3) Preparation of Antimicrobial-Containing Solution

Pipe parts were sterilized in an autoclave, and then the solutions were filled in the corresponding syringes. Then, preparation of an antimicrobial-containing solution, a triclosan dispersion, was started.

Solutions A and B were fed at a flow rate of 3 ml/min, and Solution C was fed at a flow rate of 42 ml/min by the micro syringe pumps. After 1 minute from the start of the feeding, a proper amount of the antimicrobial-containing solution, a triclosan dispersion, was inspected. The inspected antimicrobial-containing solution had a triclosan concentration of 25 mg/L, a HEPES concentration of 0.05 mol/L, and a pH of 7.

The average particle diameter of the inspected antimicrobial-containing solution was obtained by a dynamic light scattering measurement using a zeta potential & particle size analyzer (ELSZ-2 manufactured by Otsuka Electronics Co. Ltd.). The average particle diameter was 200 nm. 1 minute after the preparation (antimicrobial-containing solution 1), was increased to 2300 nm after being left stand for 2 hours (antimicrobial-containing solution2), and was further increased to 2711 nm after being left stand for 6 hours (antimicrobial-containing solution 3).

### (Evaluation of Antimicrobial Activity)

### <Test Example 1>

Evaluation of antimicrobial activity was performed using Escherichia coli NBRC3972.

One platinum loop of Escherichia coli NBRC3972 cultivated at 30°C for a night on a flat plate of soybean casein digest agar (SCDA) (manufactured by Wako Pure Chemical Industries, Ltd.) was suspended in a sterilized physiological saline to prepare an inoculum.

To 1 ml of each of antimicrobial-containing solutions 1 having triclosan concentrations adjusted to 25 mg/L, 50 mg/L, 100 mg/L, and 400 mg/L, 10 µL of the inoculum was added to incubate the both solutions for 10 minutes. The same test was performed on an antimicrobial-containing solution 2, and a dissolved triclosan solution prepared by dissolving triclosan in 100% polypropylene glycol, and diluting the obtained solution with Solution C (hereinafter referred to as a comparative solution).

The antimicrobial activity was compared based on a log reduction value of a bacterial count after the incubation. Specifically, the test solution after the incubation was inactivated by diluting with lecithin polysorbate, and the inactivated test solution was applied to soybean casein digest-lecithin polysorbate agar, and was cultivated at 30°C for a night. Then, the number of obtained colonies was counted to obtain a viable cell count after the test. Further, the inoculum was diluted, was applied to SCDA, and was cultivated at 30°C for a night. Then, the number of obtained colonies was counted to obtain an initial bacterial count. A common logarithm of surviving bacterial count relative to the initial bacterial count was considered as Log reduction value of the bacterial count. Table 5 shows the test results.

**[Table 5]**

| Triclosan concentration mg/L | Antimicrobial-containing solution 1 Average particle diameter,: 200 nm | Antimicrobial-containing solution 2 Average particle diameter: 2300 nm | Comparative solution dissolved |
|---|---|---|---|
| 25 | 3.49 | 1.37 | 0.00 |
| 50 | 4.65 | 5.43 | 0.00 |
| 100 | 5.43< | 5.43< | 0.11 |
| 400 | 5.43< | 5.43< | -* |

| | | | |
|---|---|---|---|
| Values are Log reduction values of bacterial count (-log N/N₀ c.f.u./ml, where N is a bacterial count after the incubation test, and No indicates an initial bacterial count). Symbol * indicates "no results." Symbol < indicates that the obtained value exceeded the indicated value (a detection threshold). | | | |

The results indicate that the antimicrobial-containing solutions 1 having the triclosan concentrations of 25 mg/L, 50 mg/L, and 100 mg/L showed significant antimicrobial activity as compared with the comparative solution.

The antimicrobial-containing solution 2 having the triclosan concentration of 25 mg/L showed greater antimicrobial activity than the comparative solution, and the antimicrobial-containing solutions 2 having the triclosan concentrations of 50 mg/L, and 100 mg/L showed significant antimicrobial activity as compared with the comparative solution.

### <Test Example 2>

Evaluation of the antimicrobial activity was performed using Staphylococcus aureus NBRC13276.

An inoculum was prepared in the same manner as Test Example 1 using Staphylococcus aureus. To 1 mL of each of antimicrobial-containing solutions 1 having triclosan concentrations adjusted to 25 mg/L, 200 mg/L, and 400 mg/L, 10 µL of the inoculum was added to incubate the both solutions for 10 minutes. The same test was performed on the antimicrobial-containing solutions 2, and the comparative solution. Comparison of the antimicrobial activity was performed based on the Log reduction value of the bacterial count after the incubation. Table 6 shows the test results.

**[Table 6]**

| Triclosan concentration mg/L | Antimicrobial-containing solution 1 Average particle diameter: Average particle diameter: 200 nm | Antimicrobial-containing solution 2 2300 nm | Comparative solution dissolved |
|---|---|---|---|
| 25 | 4.70< | 2.65< | 0.12 |
| 200 | 4.70< | 4.70< | -* |
| 400 | 4.70< | 4.70< | 0.17 |

| | | | |
|---|---|---|---|
| Values are Log reduction values of bacterial count (-log N/N₀ c.f.u./ml, where N is a bacterial count after the incubation test, and No indicates an initial bacterial count). Symbol * indicates "no results." Symbol < indicates that the obtained value exceeds the indicated value (a detection threshold). | | | |

The test results indicate that the antimicrobial-containing solutions 1 having the triclosan concentrations of 25 mg/ and 400 mg/L showed significant antimicrobial activity as compared with the comparative solution.

The antimicrobial-containing solution 2 having the triclosan concentration of 25 mg/L showed greater antimicrobial activity than the comparative solution, and the antimicrobial-containing solution 2 having the triclosan concentration of 400 mg/L showed significant antimicrobial activity as compared with the comparative solution.

### <Test Example 3>

Evaluation of the antimicrobial activity was performed using Pseudomonas aeruginosa NBRC13275.

An inoculum was prepared in the same manner as Test Example 1 using Pseudomonas aeruginosa. To 1 mL of each of antimicrobial-containing solutions 1 having triclosan concentrations adjusted to 25 mg/L, 200 mg/L, and 400 mg/L, 10 µL of the inoculum was added to incubate the both solutions for 10 minutes. The same test was performed on the antimicrobial-containing solutions 2, and the comparative solution. Comparison of the antimicrobial activity was performed based on the Log reduction value of the bacterial count after the incubation. Table 7 shows the test results.

**[Table 7]**

| Triclosan concentration mg/L | Antimicrobial-containing solution 1 Average particle diameter: 200 nm | Antimicrobial-containing solution 2 Average particle diameter: 2300 nm | Comparative solution dissolved |
|---|---|---|---|
| 25 | 1.77 | 1.19 | 0.50 |
| 200 | 1.77 | 2.84 | -* |
| 400 | 4.77< | 4.77< | -* |

| | | | |
|---|---|---|---|
| Values are Log reduction values of bacterial count (-log N/N₀ c.f.u./ml, where N is a bacterial count after the incubation test, and No indicates an initial bacterial count). Symbol * indicates "no results." Symbol < indicates that the obtained value exceeds the indicated value (a detection threshold). | | | |

The test results indicate that the antimicrobial-containing solutions 1 and 2 having the triclosan concentration of 25 mg/L showed greater antimicrobial activity than the comparative solution. Further, the antimicrobial-containing solutions 1 and 2 having the triclosan concentration of 400 mg/L showed significant antimicrobial activity against

### Pseudomonas aeruginosa.

Thus, the antimicrobial-containing solutions 1 and 2 have a great antimicrobial property, and show the antimicrobial activity against Pseudomonas aeruginosa.

### <Test Example 4>

Evaluation of antifungal activity was performed using Penicillium citrinum NBRC 6532.

An inoculum was prepared in the same manner as Test Example 1 except that a spore solution of Penicilliuin citrinum was used. To 1 mL of each of the antimicrobial-containing solutions 1 having the triclosan concentrations of 25 mg/L, 100 mg/L, 200 mg/L, and 400 mg/L, 10 µL of the inoculum was added to incubate the both solutions for 10 minutes. The same test was performed on the antimicrobial-containing solutions 2, and the comparative solution. Comparison of the antimicrobial activity was performed based on the Log reduction value of the bacterial count after the incubation. Table 8 shows the test results.

**[Table 8]**

| Triclosan concentration mg/L | Antimicrobial-containing solution 1 Average particle diameter: 200 nm | Antimicrobial-containing solution 2 Average particle diameter: 2300 nm | Comparative solution dissolved |
|---|---|---|---|
| 25 | 0.40 | 0.47 | 0 |
| 100 | 1.33 | 1.12 | 0 |
| 200 | 2.23< | 2.23< | 0 |
| 400 | 2.23< | 2.23< | 0 |

| | | | |
|---|---|---|---|
| Values are Log reduction values of bacterial count (-log N/N₀ c.f.u./ml, where N is a bacterial count after the incubation test, and No indicates an initial bacterial count). Symbol < indicates that the obtained value exceeds the indicated value (a detection threshold). | | | |

In general, triclosan has not been presumed as effective against Penicillium citrinum, and the comparative solution did not show the antifungal activity.

In contrast, Table 8 shows that the antimicrobial-containing solutions 1 and 2 having the triclosan concentration of 25 mg/L showed the antifungal activity against Penicillium citrinum. Further, the antimicrobial-containing solutions 1 and 2 having the triclosan concentrations of 200 mg/L and 400 mg/L showed significant antifungal activity against Penicillium citrinum.

Thus, the antimicrobial-containing solutions 1 and 2 have a good antifungal action.

### <Test Example 5>

An antimicrobial property test for evaluating the antimicrobial activity against various bacteria was performed using the antimicrobial-containing solution 1 and the comparative solution. The bacteria used were Cladosporium cladosporioides NBRC6348, Penicillium citrinum NBRC6352, Candida albicans NBRC1061, Alcaligenes faecalis NBRC13111, Klebsiella pneumoniae NBRC14940, Proteus vulgaris NBRC3167, Pseudomonas aeruginosa NBRC13275, Pseudomonas putida NBRC14164, Staphylococcus aureus NBRC13276, Serratia marcescense NBRC12648, Bacillus cereus (isolate), Bacillus coagulans (isolate)), and Escherichia coli NBRC3972.

The NBRC strains were purchased from the NITE biological research center. Inoculums were prepared in the same manner as Test Example 1 except that Alcaligenes faecalis NBRC13111, Klebsiella pneumoniae NBRC14940, Proteus vulgaris NBRC3167, Pseudomonas aeruginosa NBRC13275, Pseudomonas putida NBRC14164, Staphylococcus aureus NBRC 13276, and Serratia marcescence NBRC12648 were used. Cladosporium cladosporioides NBRC6348 and Penicillium citrinum NBRC6352 were cultivated on a flat potato dextrose agar (PDA) medium (manufactured by DIFCO) at 25°C for 1 week, and then the spore solutions were prepared using Tween 80 (Tokyo Chemical Industry Co., Ltd.) as inoculums. Candida albicans was cultivated on a flat glucose peptone agar (GPA) medium (manufactured by Wako Pure Chemical Industries Ltd.) at 30°C for 3 days, and then an inoculum was prepared in the same manner as Test Example 1.

To 1 mL of each of the antimicrobial-containing solutions 1 having the triclosan concentrations adjusted to 25 mg/L, 50 mg/L, 100 mg/L, and 400 mg/L, 10 µL of each of the inoculums was added to incubate the both solutions for 10 minutes. The same test was performed on the comparative solution. Comparison of the triclosan concentration (mg/L) at which the Log reduction value of the bacterial count after the incubation reached 1 or higher was performed. Table 9 shows the test results.

**[Table 9]**

| | Antimicrobial-containing solution 1 Average particle diameter: 200 nm | Comparative solution dissolved |
|---|---|---|
| Cladosporium cladosporioides NBRC6348 | 25 | 400< |
| Penicillium citrinum NBRC6352 | 100 | 400< |
| Candida albicans NBRC1061 | 25 | 400 |
| Alcaligenes faecalis NBRC13111 | 25 | 400 |
| Klebsiella pneumoniae NBRC14940 | 25 | 400 |
| Proteus vulgaris NBRC3167 | 25 | 100 |
| Pseudomonas aeruginosa NBRC13275 | 200 | 400< |
| Pseudomonas putida NBRC14164 | 25 | 400 |
| Staphylococcus aureus NBRC13276 | 25 | 400< |
| Serratia marcescense NBRC12648 | 200 | 400 |
| Bacillus cereus (isolate) | 25 | 100 |
| Bacillus coagulans (isolate) | 25 | 100 |
| Escherichia coli NBRC3972 | 25 | 400 |

| | | |
|---|---|---|
| Symbol < indicates that the Log reduction value of bacterial count was lower than 1 even at a concentration of 400 mg/L | | |

The results indicate that the antimicrobial-containing solution 1 (average particle diameter: 200 nm) showed significant antimicrobial activity against all the bacteria listed above as compared with the comparative solution.

### <Test Example 6>

A test was performed to check the dependence of an antimicrobial property of triclosan on a particle diameter.

One platinum loop of Escherichia coli NBRC3972 cultivated on a flat plate of soybean casein digest agar (SCDA)) (manufactured by Wako Pure Chemical Industries, Ltd.) at 30°C for a night was suspended in sterilized physiological saline to prepare an inoculum.

To 1 mL of the antimicrobial-containing solution 1 having a triclosan concentration adjusted to 25 mg/L, 10 µL of the inoculum was added to incubate the both solutions for 10 minutes. The same test was performed on the antimicrobial-containing solutions 2 and 3. Comparison of the antimicrobial activity was performed based on the Log reduction value of the bacterial count after the incubation. Table 10 shows the test results.

**[Table 10]**

| | Triclosan average particle diameter: nm | Log reduction value of bacterial count |
|---|---|---|
| Antimicrobial -containing solution 1 | 200 | 4.70< |
| Antimicrobial-containing solution 2 | 2300 | 2.65< |
| Antimicrobial-containing solution 3 | 2711 | 1.75 |

| | | |
|---|---|---|
| Values are Log reduction values of bacterial count (-log N/N₀ c.f.u./ml, where N is a bacterial count after the incubation test, and N₀ indicates an initial bacterial count). Symbol < indicates that the obtained value exceeds the indicated value (a detection threshold). | | |

This indicates that triclosan showed the antimicrobial activity irrespective of the average particle diameter, and that the antimicrobial-containing solution 1 containing triclosan having the average particle diameter of 200 nm showed significant antimicrobial activity.

As described above, the antimicrobial-containing solutions 1-3 show good antimicrobial and antifungal activity against various Gram-negative bacteria, Gram-positive bacteria, and fungi as compared with the case where triclosan is used in a solubilized state in an organic solvent or a surfactant. Further, the antimicrobial-containing solutions 1-3 show the antimicrobial activity against Pseudomonas aeruginosa, Penicillium citrinum, Cladosporium cladosporioides, and Staphylococcus aureus, for which triclosan has not been considered as effective, and show the antifungal activity against some types of mold.

### INDUSTRIAL APPLICABILITY

The present invention is useful for a method for producing an antimicrobial-containing solution which is added to detergents, stain-proofing agents, and deodorants for household, medical, and industrial applications, toiletry, cosmetics, sanitary products, etc.

### DESCRIPTION OF REFERENCE CHARACTERS

- 10: Mixer
- 11: Straight part
- 12: Branch part
- 20: Container
- 21: First chamber
- 21a, 22a: Nozzle (solution discharging part)
- 22: Second chamber
- 23: Divider

## Claims

1. A method for producing an antimicrobial-containing solution comprising:
mixing an alkaline aqueous solution which dissolves a phenol-based antimicrobial, and has a pH of 9 or higher and acid which has a pH of 6 or lower.

2. The method for producing an antimicrobial-containing solution of claim 1,
wherein
the antimicrobial-containing solution contains fine particles of the phenol-based antimicrobial having an average particle diameter of 50-5000 nm obtained by a dynamic light scattering measurement.

3. The method for producing an antimicrobial-containing solution of claim 1 or 2,
wherein
a concentration of the phenol-based antimicrobial in the alkaline aqueous solution is 10-5000 mg/L.

4. The method for producing an antimicrobial-containing solution of any one of claims 1 to 3, wherein
the alkaline aqueous solution and the acid are mixed in a volume mixing ratio of alkaline aqueous solution/acid = 1/400-100/1.

5. The method for producing an antimicrobial-containing solution of any one of claims 1 to 4, wherein
the alkaline aqueous solution and the acid are mixed by continuous mixing.

6. The method for producing an antimicrobial-containing solution of any one of claims 1 to 5, wherein
the phenol-based antimicrobial is at least one of a chlorophenol-based antimicrobial, paraben, or isopropylmethylphenol.

7. The method for producing an antimicrobial-containing solution of any one of claims 1 to 6, wherein
the phenol-based antimicrobial is triclosan.

8. The method for producing an antimicrobial-containing solution of claim 7,
wherein
a concentration of triclosan as the phenol-based antimicrobial in the antimicrobial-containing solution is 8-200 mg/L.

9. The method for producing an antimicrobial-containing solution of any one of claims 1 to 8, wherein
the acid is an acidic buffer solution.

10. A method for producing an antimicrobial-containing solution containing fine particles of a phenol-based antimicrobial having an average particle diameter of 50-5000 nm obtained by a dynamic light scattering measurement, the method comprising:
placing an alkaline aqueous solution which dissolves a phenol-based antimicrobial, and has pH of 9 or higher in a first chamber of a container having a solution discharging part, and placing acid having a pH or 6 or lower in a second chamber of the container having a solution discharging part;
simultaneously discharging the alkaline aqueous solution from the solution discharging part of the first chamber of the container, and the acid from the solution discharging part of the second chamber of the container to mix the alkaline aqueous solution and the acid outside the container.
